# EUROPEAN PATENT SPECIFICATION

(11) **EP 3 667 194 B1**
(45) Date of publication and mention of the grant of the patent: **20.10.2021**
(21) Application number: 18382848.2
(22) Date of filing: 23.11.2018
(51) Int. Cl.: F24F 13/20, F24F 13/08, G01N 33/00

(54) **AMBIENT AIR QUALITY MONITORING HOUSING**
UMGEBUNGSLUFTQUALITÄTSÜBERWACHUNGSGEHÄUSE
BOÎTIER DE SURVEILLANCE DE LA QUALITÉ DE L'AIR AMBIANT

(43) Date of publication of application: 17.06.2020
(73) Proprietor: SUEZ Groupe, 92040 Paris La Défense (FR)
(72) Inventor: TORRES JEREZ, Agustín, 28037 Madrid (ES)
(74) Representative: Elzaburu S.L.P.

(56) References cited:
- EP-A2- 2 700 925
- EP-A2- 3 258 241
- GB-A- 2 539 449
- US-A1- 2017 276 592

## Description

### Field of the invention

The present invention relates to a protective housing for an ambient air quality monitoring unit. The protective housing is intended to anchor and protect the monitoring unit to a vehicle in motion allowing to take a measurement of the quality of the ambient air.

### Background of the invention

Commercial sensors for the measurement of air quality parameters are known in the state of the art. Said sensors are usually installed in static units covered by a roof and are placed in several locations in the cities to measure the quality of ambient air.

Commercial sensing units comprise a sensing region located in at least one of the faces of the sensing unit which comprises at least a gas sensor and may also comprise particle sensors and temperature and humidity sensors. Some of the known gas sensors comprise a gas permeable membrane that is located over the face of the sensing unit and may additionally comprises a measurement electrode parallel the membrane that performs the measurement when being excited by the gas trespassing the membrane.

It is also known the need to quickly detect changes in gas concentration so as to the local or central administration may take appropriate measurements to maintain an appropriate quality of the ambient air. According to that and in order to increase the detection traceability and speed it would be advisable to have mobile measurement units.

However, directly locating known air quality monitoring units on the outside of vehicles is not recommended as these units are not prepared for this. This could lead to problems in the representation of the measurements of the volume of air to be characterized as the vehicle advances, in addition to other drawbacks such as possible entry of rainwater, pressure changes in the membranes of the sensors or possible effect of solar radiation and of the temperature of the sensors among other aspects that may alter the measurements.

It is known document EP3258241 which forms the basis for the preamble of claim 1, disclosing a particulate matter sensor device comprising an enclosure that comprises a flow inlet, a flow outlet and a flow channel extending therebetween, a radiation source for emitting radiation into the flow channel for interaction of the radiation with the particulate matter in the flow of an aerosol sample when guided through the flow channel, a radiation detector for detecting at least part of said radiation after interaction with the particulate matter. The sensor device comprises a flow modifying device arranged upstream of the radiation detector and/or of the radiation source for modifying the flow for reducing particulate matter precipitation onto the radiation detector and/or onto the radiation source and/or the channel wall sections in close proximity to the detector and/or source.

### Summary of the invention

The monitoring housing object of the invention is defined in claim 1 and comprises an external wall configured for enclosing a sensing unit having a sensing region. The claimed housing comprises:
- an airflow inlet configured for receiving incoming ambient air,
- a U-shaped path comprising:
   ∘ a straight first part of the U-shaped path, the straight first part being in fluid communication with the airflow inlet and configured for:
      ▪ receiving the incoming ambient air, and
      ▪ providing an airflow direction,
   ∘ a bended second part defining the bended part of the U-shaped path, being in fluid communication with the straight first part and configured for turning the airflow direction, and
   ∘ a straight third part of the U-shaped path, the straight third part being in fluid communication with the bended second part wherein the relative position of the straight third part is configured with respect to the sensing unit such that the airflow flowing through the straight third part is directed parallel to the sensing region of the sensing unit so that the sensing region is able to detect and measure particles and gases entrained in the ambient air.

For this application, straight means without curved or bend, therefore the U-shaped path is formed by two straight parts and one bended part or curved part in-between, notwithstanding this, the straight parts of the U shape may have a slight curvature or undulation. The U-shaped path substantially may comprise a U-shaped form or a U-shaped form with a certain tolerance, i.e., the straight parts may have an inclination with respect to the reference position between 1º and 45º, preferably between 10º and 45º as an inclination less than 10º would generate a direct shock of the air with the wall causing the impaction of particles.

For this application turning the airflow direction means changing the airflow direction in a circular direction wholly or partly around an axis or point. Therefore, the incoming airflow is, firstly, given a specific direction following the path of the straight first part and, secondly, said given airflow direction is turned at the bended second part.

The claimed configuration of the housing allows a laminar flow from the inlet to the exit assuring that the measurement is representative of the exterior ambient air and ensuring that the sensing region of the sensing unit measures fresh air instead of a mixed of air with previous trapped air that has been confined in the airflow path due to the turbulences of a turbulent airflow that would lead to a measurement not being representative of the current ambient air.

The bended part of the U-shaped path reduces the speed of the inlet air that enters the housing at a high speed. This reduction allows not only keeping a laminar airflow but also reducing the pressure of the air in the measurement or sensing region, as any change in the sensor membrane may distort the measurement.

The configuration of the housing also allows to achieve an indirect flow over the measurement region as the end part of the airflow is parallel to the sensing region. Said parallel flow avoids overpressures in the sensing membranes that can detract the taken values. A flow directed perpendicular to the membranes of the sensing region would create turbulence in the membranes and therefore distorted measurements. Besides, a direct inlet of the airflow to the membranes would lead to an uncontrolled flow that would directly impact on the sensors and subsequently generate turbulence in the measurement region, again leading to a non-representative measurement.

Therefore, conducting the air flow from the inlet to the sensing region through the U shape, firstly, eliminates the direct impact of the inlet air in the sensing region and secondly, it reduces the air pressure from the inlet and conducts the flow parallel to the sensing regions, i.e., parallel to the face of the sensing unit comprising said sensing region. This solves the drawback that at high vehicle speeds a direct impact of airflow in the membranes of the sensing region may lead to an alteration in the position of the measurement electrodes and therefore in the measurement itself.

Another advantage of the U-shaped configuration is that as the sensing region is not expose directly to the inlet it avoids water impinging over the sensors of the sensing unit, also reducing the possibility of the membranes to get wet.

Additionally, another advantage of the claimed housing is that it does not consume energy as it does not actively capture the air flow, for instance, by an active inlet comprising a fan for capturing the surrounding air.

The aforementioned housing may be used both for fixed sites and for mobile applications together with a GPS module for example.

According to the invention, the airflow inlet is located transversally to the longitudinal direction of the straight first part of the U-shaped path.

In a preferred embodiment, the housing comprises a U-shaped wall delimiting at least partially the U-shaped path, the U-shaped wall comprising:
- a straight first portion of the U-shaped wall,
- a second bended portion defining the bended portion of the U-shaped wall, and
- a straight third portion of the U-shaped wall.

Specifically, the first part of the U-shaped path may comprise a passage demarcated by the external wall of the housing and the straight first portion of the U-shaped wall.

The straight first portion of the U-shaped wall being inclined with respect to the external wall of the housing. The straight third part of the U-shaped path comprising a passage configured to be demarcated by the straight third portion of the U-shaped wall and a casing of the sensing unit.

The housing may comprise a deflector located in fluidic communication with the straight first part of the U-shaped path such that the deflector is configured to split the airflow from the straight first part into:
- a first flow that is directed towards the second and third parts of the U-shaped path, and
- a second flow that is directed parallel to the external wall of the housing.

The deflector may comprise a face delimiting the second part of U-shaped path having a bended shape for directing the airflow to the second and the third parts of the U-shaped path.

The deflector may comprise a second face configured for being located in communication with the second airflow and configured to be arranged parallel to the external wall of the housing.

The housing may additionally comprise an outlet for the exit of the airflow.

In a preferred embodiment the external wall comprises a front part configured for being located towards the incoming ambient air, said front part comprising a rounded shape for aerodynamic purpose.

In another preferred embodiment, the external wall comprises a rear part configured for being located rearwards to the incoming ambient air, said rear part comprising a spoiler shape for aerodynamic purpose. Additionally, the external wall may comprise a magnet or an opening configured for the insertion of a metallic cable.

Finally, the external wall may comprise a cover located in a face of the external wall, being the face configured to be parallel to the airflow direction.

### Description of the drawings

To complete the description and in order to provide for a better understanding of the invention, a set of drawings is provided. Said drawings form an integral part of the description and illustrate preferred embodiments of the invention. The drawings comprise the following figures.

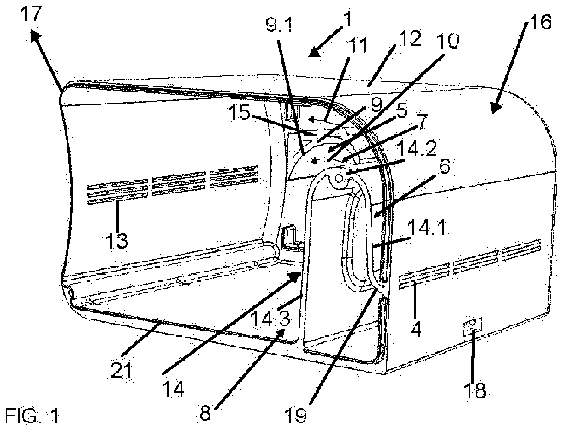

FIG. 1 shows a perspective view of an embodiment of the ambient air quality monitoring housing object of the invention.
FIG. 2A shows a lateral view of the embodiment of figure 1 also showing the sensing unit located in the housing.
FIG. 2B shows a perspective view of a schematic representation of the sensing region of the sensing unit of figure 2A.
FIG. 3 shows a perspective view of an embodiment of the ambient air quality monitoring housing showing ambient air flow lines through the housing.

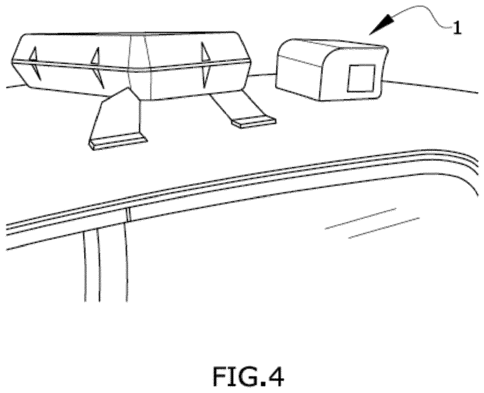

FIG. 4 shows the embodiment of figure 1 located on the roof of a vehicle.
FIG. 5 shows an exploded view of the embodiment of figure 1 together with a cover.
FIG. 6 shows a perspective view of the embodiment of figure 1 from the rear part.

### Detailed description of the invention

The figures disclose different views of an embodiment of the housing 1 object of the invention. The housing 1 comprises an external wall 12 configured for enclosing the sensing unit 2, said sensing unit 2 comprising a sensing region 3. The housing 1 comprises an airflow inlet 4 receiving incoming ambient air and a substantially U-shaped path 5 for the airflow.

The substantially U-shaped path 5 comprises:
∘ a straight first part 6 of the U-shaped path 5, the straight first part 6 being in fluid communication with the airflow inlet 4 and configured for:
   ▪ receiving the incoming ambient air, and
   ▪ providing an airflow direction,
∘ a bended second part 7 defining the bended part of the U-shaped path 5, being in fluid communication with the straight first part 6 and configured for turning the airflow direction, and
∘ a straight third part 8 of the U-shaped path 5, the straight third part 8 being in fluid communication with the bended second part 7 wherein the relative position of the straight third part 8 is configured with respect to the sensing unit 2 such that the airflow flowing through the straight third part 8 is directed parallel to the sensing region 3 of the sensing unit 2 so that the sensing region 3 is able to detect and measure particles and gases entrained in the ambient air.

More specifically, in the embodiment shown in the figures, in order to configure the above-mentioned U-shaped path, the housing comprises a U-shaped wall 14 delimiting at least partially the U-shaped path 5. The U-shaped wall 14 comprises a straight first portion 14.1 defining a first portion of the straight parts of a "U-shape", a second bended portion 14.2 defining the bended portion of a "U-shape" and a straight third portion 14.3 defining a straight second portion of the straight portions of the "U shape".

More specifically, the straight first part 6 of the U-shaped path 5 comprises a passage demarcated by the external wall 12 of the housing 1 and the straight first portion 14.1 of the U-shaped wall 14.

Additionally, in the embodiment according to the invention shown in the figures, the airflow inlet 4 is located transversally to the longitudinal direction of the first part 6 of the U-shaped path 5. This configuration has the advantage that it minimises the entrance of water in the path and also helps to reduce the speed of the incoming air into the U-shaped path 5 that reduces the possibility to have a turbulent air flow. Moreover, the first portion 14.1 of the U-shaped wall 14 is inclined with respect to the external wall 12 of the housing 1. More specifically, in the shown embodiment, the passage demarcated by the first portion 14.1 and the U-shaped wall 14 widens towards the second part 7 of the U-shaped path 5, this configuration provides an angle of inclination to avoid a frontal collision of air flow that can generate an impaction of the particles from the ambient air.

Additionally, the U-shaped wall 14 comprises a ramp 19 directed towards the inlet 4 that serves as a protection against water, allowing it to evacuate passively by gravity in periods of rain. The figures disclose a curved ramp 19 that helps to direct the inlet flow to the first part 6 of the U-shaped path 5 without impacting the U-shaped wall 14.

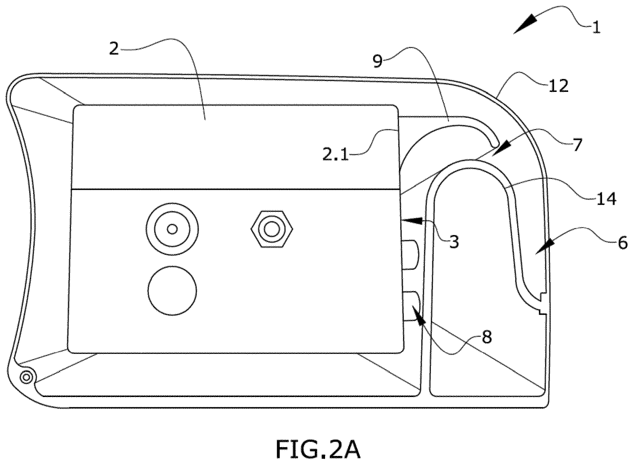

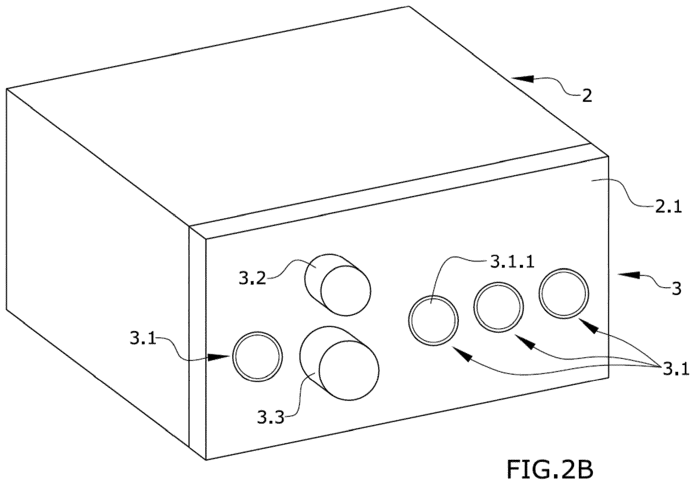

As can be seen in figure 2, the third part 8 of the U-shaped path 5 comprises a passage that is configured to be demarcated by the third portion 14.3 of the U-shaped wall 14 and a casing of the sensing unit 2 when it is located in its position within the housing 1.

In the shown embodiment, the sensing region 3 is located in one of the faces 2.1 of the sensing unit 2 and comprises several gas sensors 3.1, a particulate sensor 3.2 and an air temperature sensor 3.3. Gas sensors 3.1 comprise a membrane 3.1.1 located over the face 2.1 of the sensing unit 2 and a set of electrodes located parallel to the membrane 3.1.1. Therefore, the airflow flowing through the third part 8 is directed parallel to the sensing region 3 and hence parallel to the membranes 3.1.1. Said configuration of the third part 8 of the U-shaped path 5 with respect to the sensing region 3 avoids, for instance, the membrane 3.1.1 being curved by the force exerted by a high flow directly impinging into the membrane 3.1.1 that may curve it 3.1.1, the membrane 3.1.1 in turn pressing the electrodes and hence altering the measurement.

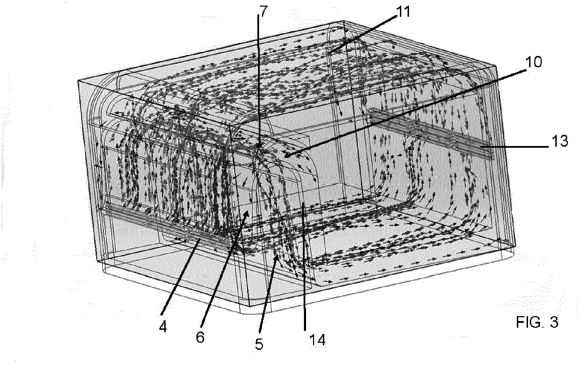

The embodiment shown in the figures additionally comprises a deflector 9 located in fluidic communication with the first part 6 of the U-shaped path 5. The deflector 9 splits the airflow coming from the first part 6 into a first flow 10 that is directed towards the second 7 and third 8 parts of the U-shaped path 5 as previously stated, and an additional second flow 11 that is directed parallel to the external wall 12 of the housing 1 as can be seen in the flow lines of figure 3.

Said additional second flow 11 improves the internal cooling of the housing 1 such that the sensing unit 2 can work within its temperature ranges. The diverted second flow 11 cools the sensing unit 2 by dragging the warm air located in the housing that has been previously exposed to heat within the housing.

Another advantage of the diverted second flow 11 is that a substantially constant first flow 10 is achieved by splitting the entrance flow. Specifically, the smallest flow goes towards the sensing region 3 and the main flow is diverted by the deflector 9. Due to said constant flow to the measuring region, the representativeness of the measurement is achieved.

The deflector 9 comprises a face 9.1 delimiting the second part 7 of U-shaped path 5 having a bended shape for directing the airflow to the second 7 and the third 8 parts of the U-shaped path 5.

The deflector 9 also comprises a second face 15 configured for being located in communication with the second airflow 11 and configured to be arranged parallel to the external wall 12 of the housing 1.

Finally, the deflector 9 of the embodiment also comprises a third face configured for being located parallel to the longitudinal direction of the passage space for supporting part of the casing of the sensing unit 2.

The monitoring housing 1 of the embodiment also comprises an outlet 13 for the exit of the airflow, in this particular case, for the exit of both first and second flows 10, 11.

As previously stated, the monitoring housing object of the invention is intended for its location on the roof of a vehicle, for this purpose the front part 16 of the external wall 12 located towards the incident ambient air comprises a rounded shape for aerodynamic purpose and additionally, the rear part 17 of the external wall 12 located rearwards the incident ambient air comprises an spoiler shape also for aerodynamic purpose.

In order to be securely joined to the vehicle, the external wall 12 of the housing 1 must be strong enough to stand the incoming air and to allow its attachment to the vehicle without affecting the data capture.

Specifically, the external wall 12 may comprise a magnet for its joining to a vehicle and/or may comprise an opening 18 configured for the insertion of a metallic cable.

In order to easily introduce and extract the sensing unit 2 and to ease maintenance and cleaning tasks, the external wall 12 comprises a cover 20 located in a face of the external wall 12 configured to be parallel to the airflow direction such that the external wall 12 is opened laterally.

Finally, the lower 21 internal face of the external wall 12 comprises an inclined surface for evacuating condensed air. In fact, locating the sensing region 3 of the sensing unit 2 parallel to the lower wall of the housing 1 wouldn't be recommended as problems with the condensed water may arises.

Water collected by the referred inclined surface is evacuated by gravity towards rear orifices 22 for its exit to the outside of the housing, preventing an internal flooding of the condensed water.

The rear outlet 13 is protected by a cover 23 whose function is to prevent the ingress of water through the openings at the rear towards the inside of the housing.

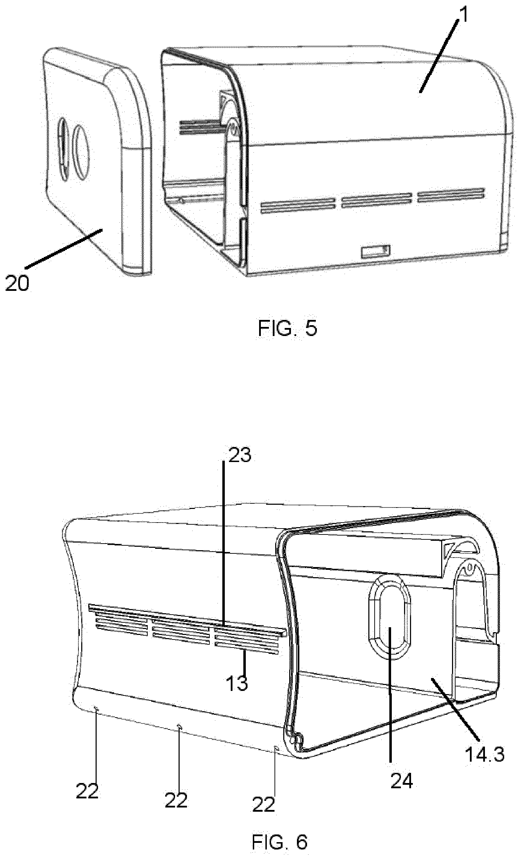

Finally, the embodiment shown in figure 6 discloses a curvature 24 made in the third part 14.3 of the U-shaped wall 14 for better accommodating the particulate matter sensor of the sensing unit 2.

## Claims

1. Ambient air quality monitoring housing (1) comprising an external wall (12) configured for enclosing a sensing unit (2) having a sensing region (3), wherein the housing (1) comprises
- an airflow inlet (4) configured for receiving incoming ambient air,
- a U-shaped path (5) comprising:
∘ a straight first part (6) of the U-shaped path (5), the straight first part (6) being in fluid communication with the airflow inlet (4) and configured for:
▪ receiving the incoming ambient air, and
▪ providing an airflow direction,
∘ a bended second part (7) defining the bended part of the U-shaped path (5), being in fluid communication with the straight first part (6) and configured for turning the airflow direction, and
∘ a straight third part (8) of the U-shaped path (5), the straight third part (8) being in fluid communication with the bended second part (7), **characterised in that** the relative position of the straight third part (8) is configured with respect to the sensing unit (2) such that the airflow flowing through the straight third part (8) is directed parallel to the sensing region (3) of the sensing unit (2) so that the sensing region (3) is able to detect and measure particles and gases entrained in the ambient air,
- and **in that** the airflow inlet (4) is located transversally to the longitudinal direction of the straight first part (6) of the U-shaped path (5).

2. Ambient air quality monitoring housing (1) according to claim 1, comprising a U-shaped wall (14) delimiting at least partially the U-shaped path (5), the U-shaped wall (14) comprising:
- a straight first portion (14.1) of the U-shaped wall (14),
- a second bended portion (14.2) defining the bended portion of the U-shaped wall (14), and
- a straight third portion (14.3) of the U-shaped wall (14).

3. Ambient air quality monitoring housing (1), according to claim 2, wherein the first part (6) of the U-shaped path (5) comprises a passage demarcated by the external wall (12) of the housing (1) and the straight first portion (14.1) of the U-shaped wall (14).

4. Ambient air quality monitoring housing (1), according to claim 3, wherein the straight first portion (14.1) of the U-shaped wall (14) is inclined with respect to the external wall (12) of the housing (1).

5. Ambient air quality monitoring housing (1), according to any preceding claim, wherein the straight third part (8) of the U-shaped path (5) comprises a passage configured to be demarcated by the straight third portion (14.3) of the U-shaped wall (14) and a casing of the sensing unit (2).

6. Ambient air quality monitoring housing (1), according to any preceding claim, comprising a deflector (9) located in fluidic communication with the straight first part (6) of the U-shaped path (5) such that the deflector (9) is configured to split the airflow from the straight first part (6) into:
- a first flow (10) that is directed towards the second (7) and third (8) parts of the U-shaped path (5), and
- a second flow (11) that is directed parallel to the external wall (12) of the housing (1).

7. Ambient air quality monitoring housing (1), according to claim 6, wherein the deflector (9) comprises a face (9.1) delimiting the second part (7) of U-shaped path (5) having a bended shape for directing the airflow to the second (7) and the third (8) parts of the U-shaped path (5).

8. Ambient air quality monitoring housing (1), according to claim 6 or 7, wherein the deflector (9) comprises a second face (15) configured for being located in communication with the second airflow (11) and configured to be arranged parallel to the external wall (12) of the housing (1).

9. Ambient air quality monitoring housing (1), according to any preceding claim, further comprising an outlet (13) for the exit of the airflow.

10. Ambient air quality monitoring housing (1), according to any preceding claim, wherein the external wall (12) comprises a front part (16) configured for being located towards the incoming ambient air, said front part (16) comprising a rounded shape for aerodynamic purpose.

11. Ambient air quality monitoring housing (1), according to any preceding claim, wherein the external wall (12) comprises a rear part (17) configured for being located rearwards to the incoming ambient air, said rear part comprising a spoiler shape for aerodynamic purpose.

12. Ambient air quality monitoring housing (1), according to any preceding claim, wherein the external wall (12) comprises a magnet.

13. Ambient air quality monitoring housing (1), according to any preceding claim, wherein the external wall (12) comprises an opening (18) configured for the insertion of a metallic cable.

14. Ambient air quality monitoring housing (1), according to any preceding claim, wherein the external wall (12) comprises a cover (20) located in a face of the external wall (12), being the face configured to be parallel to the airflow direction.

## Patentansprüche

1. Umgebungsluftqualitätsüberwachungsgehäuse (1) mit einer Außenwand (12) konfiguriert, um eine Sensoreinheit (2) mit einem Sensorbereich (3) einzuschließen, wobei das Gehäuse (1)
- einen Luftstromeinlass (4) umfasst, der konfiguriert ist, um einströmende Umgebungsluft aufzunehmen,
- einen U-förmigen Pfad (5), der Folgendes umfasst:
o einen geraden ersten Teil (6) des U-förmigen Pfads (5), wobei der gerade erste Teil (6) in Fluidverbindung mit dem Luftstromeinlass (4) steht und konfiguriert ist für:
▪ Aufnahme der einströmenden Umgebungsluft und
▪ Vorgabe einer Luftstromrichtung,
o ein gebogener zweiter Teil (7), der den gebogenen Teil des U-förmigen Pfads (5) definiert, der in Fluidverbindung mit dem geraden ersten Teil (6) steht und so konfiguriert ist, dass sie die Luftstromrichtung dreht, und
o ein gerader dritter Teil (8) des U-förmigen Pfads (5), wobei der gerade dritte Teil (8) in Fluidverbindung mit dem gebogenen zweiten Teil (7) steht, **dadurch gekennzeichnet, dass** die relative Position des geraden dritten Teils (8) in Bezug auf die Sensoreinheit (2) so konfiguriert ist, dass der durch den geraden dritten Teil (8) strömende Luftstrom parallel zum Sensorbereich (3) der Sensoreinheit (2) gerichtet ist, so dass der Sensorbereich (3) in der Lage ist, in der Umgebungsluft mitgeführte Partikel und Gase zu erfassen und zu messen,
- und dass der Luftstromeinlass (4) quer zur Längsrichtung des geraden ersten Teils (6) des U-förmigen Pfads (5) angeordnet ist.

2. Umgebungsluftqualitätsüberwachungsgehäuse (1) nach Anspruch 1, mit einer U-förmigen Wand (14), die zumindest teilweise den U-förmigen Pfad (5) begrenzt, wobei die U-förmige Wand (14) Folgendes umfasst:
- einen geraden ersten Abschnitt (14.1) der U-förmigen Wand (14),
- einen zweiten gebogenen Abschnitt (14.2), der den gebogenen Abschnitt des U-förmigen Pfads (14) definiert, und
- einen geraden dritten Abschnitt (14.3) der U-förmigen Wand (14).

3. Umgebungsluftqualitätsüberwachungsgehäuse (1) nach Anspruch 2, wobei der erste Teil (6) des U-förmigen Pfads (5) einen Durchgang umfasst, der von der Außenwand (12) des Gehäuses (1) und dem geraden ersten Abschnitt (14.1) der U-förmigen Wand (14) begrenzt wird.

4. Umgebungsluftqualitätsüberwachungsgehäuse (1) nach Anspruch 3, wobei der erste gerade Abschnitt (14.1) der U-förmigen Wand (14) in Bezug auf die Außenwand (12) des Gehäuses (1) geneigt ist.

5. Umgebungsluftqualitätsüberwachungsgehäuse (1) nach einem der vorhergehenden Ansprüche, wobei der gerade dritte Teil (8) des U-förmigen Pfads (5) einen Durchgang umfasst, der so konfiguriert ist, dass er durch den geraden dritten Abschnitt (14.3) der U-förmigen Wand (14) und ein Gehäuse der Sensoreinheit (2) abgegrenzt wird.

6. Umgebungsluftqualitätsüberwachungsgehäuse (1) nach einem der vorhergehenden Ansprüche, umfassend einen Deflektor (9), der in Fluidverbindung mit dem geraden ersten Teil (6) des U-förmigen Pfads (5) angeordnet ist, so dass der Deflektor (9) so konfiguriert ist, dass er den Luftstrom aus dem geraden ersten Teil (6) aufteilt in:
- einen ersten Strom (10), der auf den zweiten (7) und dritten (8) Teil des U-förmigen Pfads (5) gerichtet ist, und
- einen zweiten Strom (11), der parallel zur Außenwand (12) des Gehäuses (1) gerichtet ist.

7. Umgebungsluftqualitätsüberwachungsgehäuse (1) nach Anspruch 6, wobei der Deflektor (9) eine Fläche (9.1) umfasst, die den zweiten Teil (7) des U-förmigen Pfads (5) begrenzt und eine gebogene Form aufweist, um den Luftstrom auf den zweiten (7) und den dritten (8) Teil des U-förmigen Pfads (5) zu lenken.

8. Umgebungsluftqualitätsüberwachungsgehäuse (1) nach Anspruch 6 oder 7, wobei der Deflektor (9) eine zweite Fläche (15) aufweist, die so konfiguriert ist, dass sie mit dem zweiten Luftstrom (11) in Verbindung steht, und so konfiguriert ist, dass sie parallel zu der Außenwand (12) des Gehäuses (1) angeordnet ist.

9. Umgebungsluftqualitätsüberwachungsgehäuse (1) nach einem der vorhergehenden Ansprüche, ferner mit einem Auslass (13) für den Austritt des Luftstroms.

10. Umgebungsluftqualitätsüberwachungsgehäuse (1) nach einem der vorhergehenden Ansprüche, wobei die Außenwand (12) einen vorderen Teil (16) umfasst, der so konfiguriert ist, dass er der einströmenden Umgebungsluft zugewandt ist, wobei der vordere Teil (16) eine abgerundete Form für aerodynamische Zwecke aufweist.

11. Umgebungsluftqualitätsüberwachungsgehäuse (1) nach einem der vorhergehenden Ansprüche, wobei die Außenwand (12) einen hinteren Teil (17) umfasst, der so konfiguriert ist, dass er sich hinter der einströmenden Umgebungsluft befindet, wobei der hintere Teil eine Spoilerform für aerodynamische Zwecke umfasst.

12. Umgebungsluftqualitätsüberwachungsgehäuse (1) nach einem der vorhergehenden Ansprüche, wobei die Außenwand (12) einen Magneten aufweist.

13. Umgebungsluftqualitätsüberwachungsgehäuse (1) nach einem der vorhergehenden Ansprüche, wobei die Außenwand (12) eine Öffnung (18) aufweist, die für das Einführen eines Metallkabels konfiguriert ist.

14. Umgebungsluftqualitätsüberwachungsgehäuse (1) nach einem der vorhergehenden Ansprüche, wobei die Außenwand (12) eine Abdeckung (20) umfasst, die in einer Fläche der Außenwand (12) angeordnet ist, wobei die Fläche so konfiguriert ist, dass sie parallel zur Luftstromrichtung verläuft.

## Revendications

1. Boîtier de surveillance de la qualité de l'air ambiant (1) comprenant une paroi externe (12) configuré pour enfermer une unité de détection (2) ayant une région de détection (3), dans lequel le boîtier (1) comprend
- une entrée d'écoulement d'air (4) configurée pour recevoir de l'air ambiant entrant,
- un chemin en forme de U (5) comprenant :
o une première partie droite (6) du trajet en forme de U (5), la première partie droite (6) étant en communication fluidique avec l'entrée d'écoulement d'air (4) et configurée pour :
▪ recevoir l'air ambiant entrant, et
▪ fournissant une direction de flux d'air,
o une seconde partie pliée (7) définissant la partie coudée de la trajectoire en U (5), étant en communication fluidique avec la première partie droite (6) et configurée pour faire tourner la direction du flux d'air, et
o une troisième partie droite (8) de la trajectoire en U (5), la troisième partie droite (8) étant en communication fluidique avec la deuxième partie pliée (7), **caractérisé en ce que** la position relative de la troisième partie droite (8) est configurée par rapport à l'unité de détection (2) de telle sorte que le flux d'air s'écoulant à travers la troisième partie droite (8) est dirigé parallèlement à la région de détection (3) de l'unité de détection (2) de sorte que la région de détection (3) est capable de détecter et de mesurer les particules et les gaz entraînés dans l'air ambiant,
- et **en ce que** l'entrée d'écoulement d'air (4) est située transversalement à la direction longitudinale de la première partie droite (6) de la trajectoire en forme de U (5).

2. Boîtier de surveillance de la qualité de l'air ambiant (1) selon la revendication 1, comprenant une paroi en forme de U (14) délimitant au moins partiellement la trajectoire en forme de U (5), la paroi en forme de U (14) comprenant :
- une première partie droite (14.1) de la paroi en forme de U (14),
- une deuxième partie coudée (14.2) définissant la partie coudée de la paroi en forme de U (14), et
- une troisième partie droite (14.3) de la paroi en forme de U (14).

3. Boîtier de surveillance de la qualité de l'air ambiant (1), selon la revendication 2, dans lequel la première partie (6) de la trajectoire en forme de U (5) comprend un passage délimité par la paroi externe (12) du boîtier (1) et la première partie droite (14.1) de la paroi en forme de U (14).

4. Boîtier de surveillance de la qualité de l'air ambiant (1), selon la revendication 3, dans lequel la première partie droite (14.1) de la paroi en forme de U (14) est inclinée par rapport à la paroi externe (12) du boîtier (1).

5. Boîtier de surveillance de la qualité de l'air ambiant (1), selon l'une quelconque des revendications précédentes, dans lequel la troisième partie droite (8) de la trajectoire en forme de U (5) comprend un passage configuré pour être délimité par la troisième partie droite (14.3) de la paroi en forme de U (14) et un boîtier de l'unité de détection (2).

6. Boîtier de surveillance de la qualité de l'air ambiant (1), selon l'une quelconque des revendications précédentes, comprenant un déflecteur (9) situé en communication fluidique avec la première partie droite (6) de la trajectoire en forme de U (5) de telle sorte que le déflecteur (9) est configuré pour diviser le flux d'air provenant de la première partie droite (6) en :
- un premier flux (10) qui est dirigé vers les deuxième (7) et troisième (8) parties de la trajectoire en forme de U (5), et
- un deuxième flux (11) qui est dirigé parallèlement à la paroi externe (12) du boîtier (1).

7. Boîtier de surveillance de la qualité de l'air ambiant (1), selon la revendication 6, dans lequel le déflecteur (9) comprend une face (9.1) délimitant la deuxième partie (7) de la trajectoire en forme de U (5) ayant une forme courbée pour diriger le flux d'air vers les deuxième (7) et troisième (8) parties de la trajectoire en forme de U (5).

8. Boîtier de surveillance de la qualité de l'air ambiant (1), selon la revendication 6 ou 7, dans lequel le déflecteur (9) comprend une deuxième face (15) configurée pour être située en communication avec le deuxième flux d'air (11) et configurée pour être disposée parallèlement à la paroi externe (12) du boîtier (1).

9. Boîtier de surveillance de la qualité de l'air ambiant (1), selon l'une quelconque des revendications précédentes, comprenant en outre une sortie (13) pour la sortie du flux d'air.

10. Boîtier de surveillance de la qualité de l'air ambiant (1), selon l'une quelconque des revendications précédentes, dans lequel la paroi externe (12) comprend une partie avant (16) configurée pour être située vers l'air ambiant entrant, ladite partie avant (16) comprenant une forme arrondie à des fins aérodynamiques.

11. Boîtier de surveillance de la qualité de l'air ambiant (1), selon l'une quelconque des revendications précédentes, dans lequel la paroi externe (12) comprend une partie arrière (17) configurée pour être située à l'arrière de l'air ambiant entrant, ladite partie arrière comprenant une forme de spoiler à des fins aérodynamiques.

12. Boîtier de surveillance de la qualité de l'air ambiant (1), selon l'une quelconque des revendications précédentes, dans lequel la paroi externe (12) comprend un aimant.

13. Boîtier de surveillance de la qualité de l'air ambiant (1), selon l'une quelconque des revendications précédentes, dans lequel la paroi externe (12) comprend une ouverture (18) configurée pour l'insertion d'un câble métallique.

14. Boîtier de surveillance de la qualité de l'air ambiant (1), selon l'une quelconque des revendications précédentes, dans lequel la paroi externe (12) comprend un couvercle (20) situé dans une face de la paroi externe (12), la face étant configurée pour être parallèle à la direction d'écoulement de l'air.
